# EUROPEAN PATENT APPLICATION

(11) **EP 1 980 266 A1**
(43) Date of publication of application: **15.10.2008**
(21) Application number: 07106071.9
(22) Date of filing: 12.04.2007
(51) Int. Cl.: A61K 39/09

(54) **New virulence factors of Streptococcus pneumoniae**

(71) Applicant: Radboud University Nijmegen, 6500 HG Nijmegen (NL)
(72) Inventor: The designation of the inventor has not yet been filed
(74) Representative: van Loon, C.J.J.

(57) **Abstract**

The present invention provides proteins/genes, which are essential for survival, and consequently, for virulence of *Streptococcus pneumoniae in vivo,* and thus are ideal vaccine candidates for a vaccine preparation against pneumococcal infection. Further, also antibodies against said protein(s) are included in the invention.

## Description

### FIELD OF THE INVENTION

The invention relates to the field of medicine, more especially to the field of vaccines against bacterial infections, more particularly the genus *Streptococcus,* more particularly the species *Streptococcus pneumoniae.*

### BACKGROUND TO THE INVENTION

*Streptococcus pneumoniae* is the leading etiological agent of severe infections such as pneumonia, meningitis and sepsis. Young children, elderly and immunocompromised individuals are particularly vulnerable for pneumococcal diseases, which result in high morbidity and mortality (Hausdorff, W.P. et al., 2005, Lancet Infect. Dis. 5:83-93). The currently available vaccines against pneumococcal infections are based on serotype-specific capsular polysaccharides. These include a vaccine containing solely polysaccharides of 23 serotypes and a conjugate vaccine consisting of polysaccharides of the 7 most prevalent paediatric serotypes conjugated to an immunogenic carrier protein. The latter vaccine was introduced for the use in children under the age of 5, since their immune response to pure polysaccharides is inadequate. The introduction of this conjugate vaccine in the national vaccination program in the United States has had a major effect on invasive pneumococcal disease incidence (Whitney, C.G. et al., 2003, N. Eng. J. Med. 348:1737-1746).

Since at least 90 different polysaccharide structures are currently known within the species, polysaccharide-based vaccines only protect against a limited number of serotypes and hence, replacement by non-vaccine serotypes remains a threat for vaccine efficacy (Bogaert, D. et al., 2005, J. Clin. Microbiol. 43:74-83). Further, high production costs of the conjugate vaccines make their use in developing countries less feasible.

Treatment of *Streptococcus pneumoniae* infections is also impeded by the rise of strains resistant to the most commonly applied antibiotics (Levy, S.B. and Marshall, B., 2004, Nat. Med. 10:S122-S129). The development of an affordable effective vaccine against invasive pneumococcal disease in, especially, young children and elderly will have major benefits in terms of reducing disease burden and health care costs in both developed and developing countries. Immunogenic antigens of pneumococcal origin that are conserved amongst numerous serotypes would be desirable for conferring protection against infections caused by a broad range of serotypes. Much research effort is currently invested in search for pneumococcal proteins with protective potential to be included in future vaccines.

Methods searching for surface proteins of *Streptococcus pneumoniae* have been described (e.g.WO 98/18930), other methods have used immunological approaches to find possible antigenic determinants (WO 01/12219). On a genetic level, several methods have been used to determine which genes are needed by *Streptococcus pneumoniae* in the various niches it occupies in the host (conditionally essential genes) such as transcriptome analysis (Orihuela, C.J. et al., 2004, Infect. Immun. 72:4766-4777), differential fluorescence induction (Marra, A. et al., 2002, Infect. Immun. 70:1422-1433) and signature-tagged mutagenesis (Hava, D.L. and Camilli, A., 2002, Mol. Microbiol. 45:1389-1406; Lau, G.W. et al., 2001, Mol. Microbiol. 40:555-571; Polissi, A. et al., 1998, Infect. Immun. 66:5620-5629). Through these and other methods, several pneumococcal proteins have been identified and further investigated as potential vaccine candidates, such as the toxoid derivative of pneumolysin (PdB) (Briles, D.E. et al., 2003, J. Infect. Dis. 188:339-348; Ogunniyi, A.D. et al., 2000, Infect. Immun. 68:3028-3033; Ogunniyi, A.D. et al., 2001, Infect. Immun. 69:5997-6003), pneumococcal surface protein A (PspA) (Briles, D.E. et al., 2003, *supra;* Briles, D.E. et al., 2000, Infect. Immun. 68:796-800; Swiatlo, E. et al., 2003, Infect. Immun. 2003, 71:7149-7153; Wu, H.Y. et al., 1997, J. Infect. Dis. 175:839-846), pneumococcal surface adhesion A (PsaA) (Briles, D.E. et al., 2000, *supra),* choline binding protein A (CbpA) (Ogunniyi, A.D. et al., 2000, *supra),* BVH-3 (Hamel, J. et al., 2004, Infect. Immun. 72:2659-2670), PiuA and PiaA (Browns, J.S. et al., 2001, Infect. Immun. 69:6702-6706), pneumococcal protective protein A (PppA) (Green, B.A. et al., 2005, Infect. Immun. 73:981-989), putative proteinase maturation protein A (PpmA) (Adrian, P.V. et al., 2004, Vaccine 22:2737-2742; Overweg, K. et al., 2000, Infect. Immun. 68:4180-4188), IgA1 protease (IgA1p) (Weiser, J.N. et al., 2003, Proc. Natl. Acad. Sci. USA 100:4215-4220) and the streptococcal lipoprotein rotamase A (SlrA) (Adrian, P.V. et al. *supra).*

Yet, there is still need for new vaccine candidates.

### SUMMARY OF THE INVENTION

The inventors now have found several proteins/genes of *Streptococcus pneumoniae* which are essential for the virulence of the pathogen, and which thus would be applicable in a vaccine for combating pneumococcal infections.

Accordingly, the invention comprises a vaccine formulation providing protection against pneumococcal infection in a subject, said formulation comprising an effective amount of a protein encoded by a gene listed in Table 1 and/or Table 2 or a functional homologue or an immunogenic part thereof together with at least one of a pharmaceutically acceptable diluent, carrier, excipient or adjuvant therefore. Preferably said immunogenic part is antigenic determinant of said pathogen. The protein of said formulation is preferably encoded by a gene listed in Table 3, or alternatively said protein is encoded by a gene listed in Table 1A, Table 1B, Table 2A and/or Table 2B, while most preferably said protein is encoded by a gene listed in both Table 1A or Table 1B and Table 2A or Table 2B.

Further comprised in the invention is a formulation according to the above formulations, wherein said formulation provides protections against pneumonia, meningitis, otitis media and/or sepsis caused by *Streptococcus pneumoniae.*

In another embodiment, the invention comprises a protein encoded by a gene listed in Table 1 and/or 2 or an immunogenic part thereof, for use as a vaccine.

In another embodiment the invention comprises an antibody against a protein encoded by a gene listed in Table 1 and/or 2 or fragment thereof, preferably a humanized antibody or fragment thereof. Preferably said antibody or fragment thereof, preferably a humanized antibody or fragment thereof is for use as a medicament for the prophylactic or therapeutic treatment of a pneumococcal infection in a subject.

In yet another embodiment the invention comprises the use of said antibody or fragment thereof, preferably said humanized antibody or fragment thereof for the manufacture of a medicament for the prophylactic or therapeutic treatment of a pneumococcal infection in a subject.

Also comprised in the invention is a pharmaceutical composition comprising said antibody or fragment thereof, preferably said humanized antibody or fragment thereof, and a pharmaceutically acceptable carrier.

Further comprised in the invention is a method for prophylactic or therapeutic treatment of a pneumococcal infection in a subject comprising administering to a subject in need of such treatment an effective amount of a vaccine formulation as defined above and/or an effective amount of a pharmaceutical composition as defined above.
In another embodiment the invention comprises a method for preparing a pneumococcal vaccine formulation, the said method comprising bringing into association, an effective amount of a protein encoded by a gene listed in Table 1 and/or Table 2 or an immunogenic part thereof and at least one of a pharmaceutically acceptable diluent, carrier, excipient or adjuvant therefore. Preferably, said method comprises bringing into association, an effective amount of an antibody, preferably a humanized antibody, or fragment thereof, as described above and a pharmaceutically acceptable carrier.

### LEGENDS TO THE FIGURES

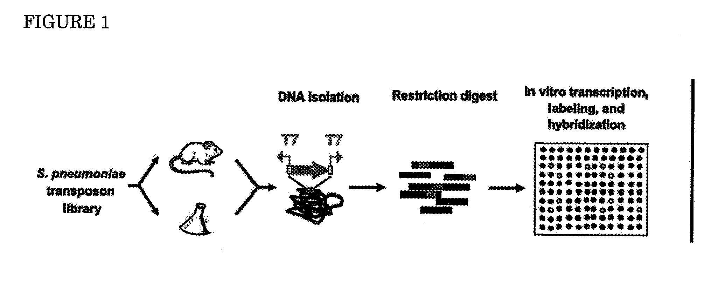

Fig. 1 shows a schematic representation of the GAF procedure. A large *Streptococcus pneumoniae* transposon library is grown under nonselective and selective conditions. Subsequently, chromosomal DNA containing transposon (grey rectangle) with outward-facing T7 RNA polymerase promoters (arrow with T7) is isolated from each population. The DNA is digested, and the DNA adjacent to the transposon insertion site is amplified using *in vitro* transcription with T7 RNA polymerase. The RNA is used in standard procedures for microarray probe synthesis. Co-hybridization of probes derived from non-selective and selective conditions to a microarray will reveal which genes were disrupted in the mutants that disappeared during selection: only material derived from the nonselective condition will hybridise to those spots (grey spots).

### DETAILED DESCRIPTION

A "virulence factor" is referred to herein as a property of a pathogen that allows it to colonize and survive in the host, and consequently to cause disease. Virulence factors may distinguish a pathogenic micro-organism from otherwise identical non-pathogenic micro-organisms by allowing pathogens to invade, adhere to, and/or colonize a host, and then harm the host, as for an organism to be pathogenic it must be able to invade a host, multiply in the host, evade host defences, and harm the host in some way. As used herein the gene product of the genes of Table 1 and 2 are virulence factors.

The terms "invade" and "invasion" refer to the growing of infections into tissues, i.e., through and then beneath epithelial tissues, in particular it encompasses to the process of passage of mucosal tissue, either in the nasopharyngeal tissue or in the lungs, to the lymph fluid, the blood and/or the meningi. Thus, it encompasses both nasopharyngeal colonization and dissemination to the blood/meningi.

The term "functional fragment" refers to a shortened version of the protein, which is a functional variant or functional derivative. A "functional variant" or a "functional derivative" of a protein is a protein the amino acid sequence of which can be derived from the amino acid sequence of the original protein by the substitution, deletion and/or addition of one or more amino acid residues in a way that, in spite of the change in the amino acid sequence, the functional variant retains at least a part of at least one of the biological activities of the original protein that is detectable for a person skilled in the art. A functional variant is generally at least 60% homologous (preferably the amino acid sequence is at least 60% identical), advantageously at least 70% homologous and even more advantageously at least 80 or 90% homologous to the protein from which it can be derived. A functional variant may also be any functional part of a protein; the function in the present case being particularly but not exclusively essential activity for nasopharyngeal or blood colonization. "Functional" as used herein means functional in *Streptococcus pneumoniae* bacteria and capable of eliciting antibodies which give protection against disease caused by said bacteria.

The expression "conservative substitutions" as used with respect to amino acids relates to the substitution of a given amino acid by an amino acid having physicochemical characteristics in the same class. Thus where an amino acid of the protein encoded by the genes listed in Tables 1 and 2 has a hydrophobic characterising group, a conservative substitution replaces it by another amino acid also having a hydrophobic characterising group; other such classes are those where the characterising group is hydrophilic, cationic, anionic or contains a thiol or thioether. Such substitutions are well known to those of ordinary skill in the art, i.e. see US 5,380,712. Conservative amino acid substitutions may be made, for example within the group of aliphatic nonpolar amino acids (Gly, Ala, Pro, Ile, Leu, Val), the group of polar uncharged amino acids (Cys, Ser, Thr, Met, Asn, Gln), the group of polar charged amino acids (Asp, Glu, Lys, Arg) or the group of aromatic amino acids (His, Phe, Tyr, Trp).

The term " immunogenic part" includes reference to any part of a protein encoded by the genes listed in Tables 1 and 2, or a functional homologue or functional fragment thereof, which is capable of eliciting an immune response in a mammal. Said immunogenic part preferably corresponds to an antigenic determinant of said pathogen.

As used herein, the term "antigen" refers to a molecule capable of being bound by an antibody or a T cell receptor (TCR) if presented by molecules of the major histocompatibility complex (MHC). The term "antigen", as used herein, also encompasses T-cell epitopes. A T-cell epitope is recognized by a T-cell receptor in the context of a MHC class I, present on all cells of the body except erythrocytes, or class II, present on immune cells and in particular antigen presenting cells. This recognition event leads to activation of T-cells and subsequent effector mechanisms such as proliferation of the T-cells, cytokine secretion, perforin secretion etc. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a T-Helper cell epitope and is given in adjuvant. An antigen can have one or more epitopes (Band T-epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens. Antigens, as used herein, include infectious disease antigens, more especially antigens of *Streptococcus pneumoniae,* more preferable antigens derived from the proteins encoded by the genes listed in Tables 1 and 2 and fragments and derivatives thereof. Furthermore, antigens used for the present invention can be peptides, proteins, domains, or lipids, especially those lipids that are associated to the proteins encoded by the genes listed in Tables 1 and 2 as lipoproteins.

As used herein, the term "antigenic determinant" is meant to refer to that portion of an antigen that is specifically recognized by either B- or T-lymphocytes. B-lymphocytes respond to foreign antigenic determinants via antibody production, whereas T-lymphocytes are the mediator of cellular immunity. Thus, antigenic determinants or epitopes are those parts of an antigen that are recognized by antibodies, or in the context of an MHC, by T-cell receptors. An antigenic determinant may contain one or more epitopes. Epitopes may be present on the intracellular (inside), transmembrane spanning (transmembrane), as well as extracellular (outside) regions of a protein molecule. It is expected that antigenic determinants are associated with in particular those regions of the surface proteins encoded by the genes listed in Tables 1A, 1B, 2A and 2B which are on the outside of the cytoplasmic membrane of the bacteria. These regions can be predicted from the sequences as provided, by using for instance one of the software programs SignalP3.0, PSORTb or TMHMM, e.g. version 2.0c, which provides a method for prediction transmembrane helices based on a hidden Markov model.

The term "prophylactic or therapeutic treatment of an infection by *Streptococcus pneumoniae"* or "prophylactic or therapeutic treatment of a pneumococcal infection" refers to both prophylactic or therapeutic treatments wherein virulence of the pathogen is blocked or diminished, but also to treatments wherein antibodies against any of the proteins encoded by the genes listed in Table 1 or 2 recognize the bacteria and will protect the host against infection, either directly through immune clearance, or indirectly by blocking the activity of the protein, thereby inhibiting the growth of the bacteria. Also, the term refers to blocking the function of any of the proteins encoded by the genes listed in Tables 1 and 2 *in vivo* thereby reducing the adhesion abilities of the pathogen with a concomitant reduction in colonization and invasion capabilities. The term thus includes inducing immune responses in subjects using vaccine formulations of the invention, as well as inhibiting growth of the pathogen *in vivo* by using antibodies of the present invention as an active compound in a pharmaceutical composition administered to the subject. Also included is the inhibition of the virulence and/or growth of the bacteria by treatment with antibiotics.

The term "antibody" refers to molecules which are capable of binding an epitope or antigenic determinant and includes reference to antigen binding forms of antibodies (e. g., Fab, F(ab)2). The term "antibody" frequently refers to a polypeptide substantially encoded by an immunoglobulin gene or immunoglobulin genes, or fragments thereof which specifically bind and recognize an analyte (antigen). However, while various antibody fragments can be defined in terms of the digestion of an intact antibody, one of skill will appreciate that such fragments may be synthesized de novo either chemically or by utilizing recombinant DNA methodology. Thus, the term antibody, as used herein, also includes antibody fragments such as single chain Fv, chimeric antibodies (i. e., comprising constant and variable regions from different species), humanized antibodies (i. e., comprising a complementarity determining region (CDR) from a non-human source) and heteroconjugate antibodies (e. g., bispecific antibodies). The antibody can be monoclonal or polyclonal and can be prepared by techniques that are well known in the art such as immunization of a host and collection of sera (polyclonal) (see, e.g., Parker, Radioimmunoassay of Biologically Active Compounds, Prentice-Hall (Englewood Cliffs, N.J., U.S., 1976), Butler, J. Immunol. Meth. 7, 1-24 (1975); Broughton and Strong, Clin, Chem. 22, 726-732 (1976); and Playfair, et al., Br. Med. Bull. 30, 24-31 (1974)) or by preparing continuous hybrid cell lines and collecting the secreted protein (monoclonal) (see, *e.g.,* Kohler et al in Nature 256, 495-497 (1975) and Eur. J. Immunol. 6, 511-519 (1976); by Milstein et al. Nature 266, 550-552 (1977); and by Walsh Nature 266, 495 (1977)) or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')2, Fab', and the like. In addition, aggregates, polymers, and conjugates of immunoglobulins or their fragments can be used where appropriate so long as binding affinity for a particular molecule is maintained.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as Fab, F(ab')2, Fv, and others, such as CDR fragments, which retain the antigen binding function of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "humanized monoclonal antibodies" means that at least a portion of the exposed amino acids in the framework regions of the antibody (or fragment), which do not match with the corresponding amino acids in the most homologous human counterparts, are changed, such as by site directed mutagenesis of the DNA encoding the antibody. Because these exposed amino acids are on the surface of the molecule, this technique is called "resurfacing." Moreover, because the amino acids on the surface of the molecule are the ones most likely to give rise to an immune response, this resurfacing decreases the immunogenicity of the monoclonal antibody when administered to a species whose cell line was not used to generate the antibody, such as a human. The term "humanized monoclonal antibody" also includes chimeric antibody wherein the light and heavy variable regions of a monoclonal antibody generated by a hybridoma from a non-human cell line are each attached, via recombinant technology, to one human light chain constant region and at least one heavy chain constant region, respectively. The preparation of such chimeric (i. e., humanized) antibodies are well known in the art.

The term "specifically recognizing", includes reference to a binding reaction between an antibody and a protein having an epitope recognized by the antigen binding site of the antibody. This binding reaction is determinative of the presence of a protein having the recognized epitope amongst the presence of a heterogeneous population of proteins and other biologics. Specific binding to an antibody under such conditions may require an antibody that is selected for its specificity for a particular protein. For example, antibodies raised to the proteins encoded by the genes listed in Tables 1 and 2 of the present invention can be selected to obtain antibodies specifically recognizing said proteins. The proteins used as immunogens can be in native conformation or denatured so as to provide a linear epitope. A variety of immunoassay formats may be used to select antibodies specifically recognizing a particular protein (or other analyte). For example, solid-phase ELISA immunoassays are routinely used to select monoclonal antibodies specifically immunoreactive with a protein. See Harlow and Lane, Antibodies, A Laboratory Manual, Cold Spring Harbor Publications, New York (1988), for a description of immunoassay formats and conditions that can be used to determine selective reactivity.

A "subject" as referred to herein is meant to include mammals and other animals, wherein mammals include for example, humans, apes, monkeys, horses, cattle, pigs, goats, dogs, cats, rats, mice, and sheep. The term "non-human animal" is meant to disclaim humans. Preferably in the present invention, the subject is a human, more preferably a child or an elderly person.

The virulence proteins/genes of the present invention have been identified by genomic array footprinting (GAF), which is a high-throughput method to identify conditionally essential gene in *Streptococcus pneumoniae* by using a combination of random transposon mutagenesis and microarray technology (see Bijlsma, J.J.E. et al., 2007, Appl. Environm. Microbiol. 73(5):1514-1524). GAF detects the transposon insertion sites in a mutant library by amplifying and labelling the chromosomal DNA adjacent to the transposon and subsequent hybridisation of these probes to a microarray. Identification of transposon insertion sites in mutants that have disappeared from the library due to selection, which represent conditionally essential genes, is achieved by differential hybridisation of the probes generated from the library grown under two conditions to an array (Fig. 1). For the detection of essential genes for nasopharyngeal colonization and/or dissemination to the blood mutant libraries of *Streptococcus pneumoniae* (prepared as described in the experimental part) were used to infect mice in a murine pneumonia model of infection. After challenge mutants were identified that had disappeared from the nasopharyngeal lavage and/or blood samples taken from the mice, and the disrupted genes of these mutants were identified.

The genes found to be essential for nasopharyngeal colonization are provided in Table 1. Table 1A lists the genes which are predicted to be located at the surface based on their sequence (using various prediction servers, such as SignalP3.0 (http://www.cbs.dtu.dk/services/SignalP). PSORTb (http://www.psort.org) and TMHMM (http://www.cbs.dtu.dk/services/TMHMM)), Table 1B lists the genes which are predicted to be surface localised, based on the following criteria:
- at least one predicted transmembrane helix; or
- components IIC and IID of PTS systems; or
- capsule gene loci.
Table 1C lists the genes which are predicted to be localised in the cytoplasma, or which could not be used in prediction servers, because the ORF was annotated as pseudogene or frame-shifted.

The genes found to be essential for dissemination to the blood are listed in Tables 2A-2C on the same criteria as for Table 1. The surface-localised proteins of the genes of Table 1 and Table 2 are especially preferred as a vaccine component according to the present invention.

Table 3 lists the genes which appear both in Table 1 and Table 2 and which are especially preferred in the present invention.

Next to the genes listed in Tables 1 and 2, more genes (listed in Table 4) have been identified in the current experimental set-up. These genes/proteins of Table 4 have been identified earlier as genes/proteins which would be suitable as vaccine candidates for *streptococcus pneumoniae.* The fact that these genes were found in our experiments emphasizes the usefulness of the methodology for finding potential vaccine candidates.

**Table 1A. Conditionally essential Streptococcus pneumoniae genes identified in nasopharyngeal lavage that encode a predicted surface-localised protein. Locus indicates the gene number assigned by TIGR-CMG annotation (TIGR Comprehensive Microbial Resource database http://cmr.tigr.org/tigr-scripts/CMR/CmrHomePage.cgi).**

| **Locus Annotation** | **Gene** | **Mainrole** |
|---|---|---|
| SP0042 competence factor transporting ATP-binding/permease protein ComA | comA | Cellular processes |
| SP0112 amino acid ABC transporter, periplasmic AA-binding protein, putative | | Transport and binding proteins |
| SP0749 branched-chain amino acid ABC transporter, AA-binding protein | livJ | Transport and binding proteins |
| SP0769 VanZF-related protein | | Unknown function |
| SP1002 adhesion lipoprotein | Imb | Cellular processes |
| SP1437 conserved domain protein | | Hypothetical proteins-Domain |
| SP1492 cell wall surface anchor family protein | | Cell envelope |
| SP1624 acyltransferase family protein | | Unknown function |
| SP1690 ABC transporter, substrate-binding protein | | Transport and binding proteins |
| SP1826 ABC transporter, substrate-binding protein | | Transport and binding proteins |
| SP1955 hypothetical protein | | Hypothetical proteins |
| SP2010 penicillin-binding protein 2A | pbp2A | Cell envelope |
| SP2050 competence protein CgID | cgID | Cellular processes |
| SP2084 phosphate ABC transporter, phosphate-binding protein | pstS | Transport and binding proteins |
| SP2147 hypothetical protein | | Hypothetical proteins |
| SP2197 ABC transporter, substrate-binding protein, putative | | Transport and binding proteins |

**Table 1B. Conditionally essential Streptococcus pneumoniae genes identified in nasopharyngeal lavage, which encode a predicted surface-localised protein.**

| **Locus Annotation** | **Gene** | **Mainrole** |
|---|---|---|
| SP0282 PTS system, mannose-specific IID component | | Transport and binding proteins |
| SP0283 PTS system, mannose-specific IIC component | manM | Transport and binding proteins |
| SP0514 hypothetical protein | | Hypothetical proteins |
| SP0866 hypothetical protein | | Hypothetical proteins |
| SP1368 psr protein | | Unknown function |
| SP1617 PTS system, IIC component | | Transport and binding proteins |
| SP2038 PTS system, membrane component, putative | | Transport and binding proteins |
| SP2205 DHH subfamily 1 protein | | Unknown function |

**Table 1C. Conditionally essential Streptococcus pneumoniae genes identified in nasopharyngeal lavage, which encode a predicted cytoplasm-localised protein.**

| **SP nr. Annotation** | **Gene** | **Mainrole** |
|---|---|---|
| SP0021 deoxyuridine 5triphosphate nucleotidohydrolase, putative | | Purines, pyrimidines, etc |
| SP0051 phosphoribosylamine--glycine ligase | purD | Purines, pyrimidines, etc |
| SP0058 transcriptional regulator, GntR family | | Regulatory functions |
| SP0061 PTS system, IIB component | | Transport and binding proteins |
| SP0106 L-serine dehydratase, iron-sulfur-dependent, beta subunit | sdhB | Energy metabolism |
| SP0113 argininosuccinate synthase, truncation | argG | Disrupted reading frame |
| SP0116 hypothetical protein | | Hypothetical proteins |
| SP0152 ABC transporter, permease protein, putative | | Transport and binding proteins |
| SP0168 macrolide efflux protein, putative | | Transport and binding proteins |
| SP0178 riboflavin biosynthesis protein RibD | ribD | Biosynthesis of cofactors, etc |
| SP0181 conserved hypothetical protein | | Hypothetical proteins-Conserved |
| SP0182 MccC family protein | | Unknown function |
| SP0200 competence-induced protein Ccs4 | ccs4 | Unknown function |
| SP0202 anaerobic ribonucleoside-triphosphate reductase | nrdD | Purines, pyrimidines, etc |
| SP0206 hypothetical protein | | Hypothetical proteins |
| SP0245 pyruvate formate-lyase-activating enzyme, putative | | Energy metabolism |
| SP0255 acetyltransferase, GNAT family | | Unknown function |
| SP0259 Holliday junction DNA helicase RuvB | ruvB | DNA metabolism |
| SP0263 eep protein | eep | Cellular processes |
| SP0280 ribosomal small subunit pseudouridine synthase A | rsuA-1 | Protein synthesis |
| SP0302 conserved domain protein | | Hypothetical proteins-Domain |
| SP0317 4-hydroxy-2-oxoglutarate aldolase/2-deydro-3-deoxyphosphogluconate aldolase | | Energy metabolism |
| SP0318 carbohydrate kinase, PfkB family | | Energy metabolism |
| SP0321 PTS system, IIA component | | Transport and binding proteins |
| SP0333 transcriptional regulator, putative | | Regulatory functions |
| SP0340 autoinducer-2 production protein | luxS | Cellular processes |
| SP0404 hypothetical protein | | Hypothetical proteins |
| SP0409 conserved hypothetical protein | | Hypothetical proteins-Conserved |
| SP0442 conserved hypothetical protein | | Hypothetical proteins-Conserved |
| SP0446 acetolactate synthase, small subunit | ilvN | Amino acid biosynthesis |
| SP0453 amino acid ABC transporter, AA-binding protein/permease protein | | Transport and binding proteins |
| SP0473 ROK family protein | | Regulatory functions |
| SP0481 conserved hypothetical protein | | Hypothetical proteins-Conserved |
| SP0482 conserved hypothetical protein | | Hypothetical proteins-Conserved |
| SP0488 conserved hypothetical protein | | Hypothetical proteins-Conserved |
| SP0489 PAP2 family protein | | Unknown function |
| SP0491 hypothetical protein | | Hypothetical proteins |
| SP0515 heat-inducible transcription repressor HrcA | hrcA | Regulatory functions |
| SP0521 HIT family protein | | Unknown function |
| SP0525 blpS protein | blpS | Unknown function |
| SP0557 ribosome-binding factor A | rbfA | Transcription |
| SP0565 conserved domain protein | | Hypothetical proteins-Domain |
| SP0585 5-methyltetrahydropteroyltriglutamate-homocysteine methyltransferase | metE | Amino acid biosynthesis |
| SP0592 conserved hypothetical protein | | Hypothetical proteins-Conserved |
| SP0611 single-stranded-DNA-specific exonuclease RecJ | recJ | DNA metabolism |
| SP0615 beta-lactam resistance factor | fibA | Cell envelope |
| SP0634 conserved domain protein | | Hypothetical proteins-Domain |
| SP0676 transcriptional regulator, putative | | Regulatory functions |
| SP0687 ABC transporter, ATP-binding protein | | Transport and binding proteins |
| SP0695 HesA/MoeB/ThiF family protein | | Unknown function |
| SP0696 hypothetical protein | | Hypothetical proteins |
| SP0705 hypothetical protein | | Hypothetical proteins |
| SP0736 mannose-6-phosphate isomerase | manA | Energy metabolism |
| SP0743 transcriptional regulator, TetR family | | Regulatory functions |
| SP0744 cytidine and deoxycytidylate deaminase family protein | | Unknown function |
| SP0745 uracil phosphoribosyltransferase | upp | Purines, pyrimidines, etc |
| SP0748 conserved hypothetical protein | | Hypothetical proteins-Conserved |
| SP0750 branched-chain amino acid ABC transporter, permease protein | livH | Transport and binding proteins |
| SP0751 branched-chain amino acid ABC transporter, permease protein | livM | Transport and binding proteins |
| SP0752 branched-chain amino acid ABC transporter, ATP-binding protein | livG | Transport and binding proteins |
| SP0753 branched-chain amino acid ABC transporter, ATP-binding protein | livF | Transport and binding proteins |
| SP0754 acetoin utilization protein AcuB, putative | | Energy metabolism |
| SP0768 conserved hypothetical protein | | Hypothetical proteins-Conserved |
| SP0777 hypothetical protein | | Hypothetical proteins |
| SP0786 ABC transporter, ATP-binding protein | | Transport and binding proteins |
| SP0815 hypothetical protein | | Hypothetical proteins |
| SP0816 hypothetical protein | | Hypothetical proteins |
| SP0817 MutT/nudix family protein | | DNA metabolism |
| SP0828 ribose 5-phosphate isomerase | rpiA | Energy metabolism |
| SP0830 hypothetical protein | | Hypothetical proteins |
| SP0831 purine nucleoside phosphorylase, family 2 | deoD | Purines, pyrimidines, etc |
| SP0848 sugar ABC transporter, permease protein, putative | | Transport and binding proteins |
| SP0876 1-phosphofructokinase, putative | | Energy metabolism |
| SP0881 thiazole biosynthesis protein Thil | thil | Biosynthesis of cofactors, etc |
| SP0882 conserved hypothetical protein | | Hypothetical proteins-Conserved |
| SP0893 transcriptional repressor, putative | | Regulatory functions |
| SP0921 conserved hypothetical protein | | Hypothetical proteins-Conserved |
| SP0950 acetyltransferase, GNAT family | | Unknown function |
| SP0962 lactoylglutathione lyase | gloA | Energy metabolism |
| SP0972 multi-drug resistance efflux pump | pmrA | Transport and binding proteins |
| SP1011 GtrA family protein | | Unknown function |
| SP1025 hypothetical protein | | Hypothetical proteins |
| SP1039 hypothetical protein | | Hypothetical proteins |
| SP1052 phosphoesterase, putative | | Unknown function |
| SP1053 conserved domain protein | | Hypothetical proteins-Domain |
| SP1054 Tn5252, Orf 10 protein | | Mobile and extrachromosomal ele |
| SP1055 Tn5252, Orf 9 protein | | Mobile and extrachromosomal ele |
| SP1061 protein kinase, putative | | Regulatory functions |
| SP1096 conserved hypothetical protein | | Hypothetical proteins-Conserved |
| SP1104 hypothetical protein | | Cell envelope |
| SP1119 glyceraldehyde-3-phosphate dehydrogenase, NADP-dependent | gapN | Energy metabolism |
| SP1136 conserved domain protein | | Hypothetical proteins-Domain |
| SP1167 dihydroorotase, multifunctional complex type | pyrC | Purines, pyrimidines, etc |
| SP1177 phosphocarrier protein HPr | ptsH | Transport and binding proteins |
| SP1190 tagatose 1,6-diphosphate aldolase | lacD | Energy metabolism |
| SP1222 type II restriction endonuclease, putative | | DNA metabolism |
| SP1235 MutT/nudix family protein | | DNA metabolism |
| SP1245 Cof family protein | | Unknown function |
| SP1280 conserved hypothetical protein | | Hypothetical proteins-Conserved |
| SP1289 hypothetical protein | | Hypothetical proteins |
| SP1298 DHH subfamily 1 protein | | Unknown function |
| SP1299 ribosomal protein L31 | rpmE | Protein synthesis |
| SP1320 v-type sodium ATP synthase, subunit E | ntpE | Transport and binding proteins |
| SP1333 hypothetical protein | | Hypothetical proteins |
| SP1341 ABC transporter, ATP-binding protein | | Transport and binding proteins |
| SP1370 shikimate kinase | aroK | Amino acid biosynthesis |
| SP1371 3-phosphoshikimate 1-carboxyvinyltransferase | aroA | Amino acid biosynthesis |
| SP1375 3-dehydroquinate synthase | aroB | Amino acid biosynthesis |
| SP1376 shikimate 5-dehydrogenase | aroE | Amino acid biosynthesis |
| SP1393 conserved hypothetical protein | | Hypothetical proteins-Conserved |
| SP1397 phosphate ABC transporter, ATP-binding protein, putative | | Transport and binding proteins |
| SP1422 hypothetical protein | | Hypothetical proteins |
| SP1429 peptidase, U32 family | | Protein fate |
| SP1449 cppA protein | cppA | Unknown function |
| SP1463 methylated-DNA-protein-cysteine S-methyltransferase | ogt | DNA metabolism |
| SP1465 hypothetical protein | | Hypothetical proteins |
| SP1467 conserved hypothetical protein | | Hypothetical proteins-Conserved |
| SP1468 pyridoxine biosynthesis protein | | Biosynthesis of cofactors, etc |
| SP1471 oxidoreductase, putative | | Unknown function |
| SP1478 oxidoreductase, aldo/keto reductase family | | Unknown function |
| SP1506 conserved hypothetical protein | | Hypothetical proteins-Conserved |
| SP1519 acetyltransferase, GNAT family | | Unknown function |
| SP1537 general stress protein 13, putative | | Cellular processes |
| SP1562 hypothetical protein | | Hypothetical proteins |
| SP1563 pyridine nucleotide-disulphide oxidoreductase family protein | | Unknown function |
| SP1567 endoribonuclease L-PSP | | Transcription |
| SP1568 GTP-binding protein | | Unknown function |
| SP1578 methyltransferase, putative | | Unknown function |
| SP1601 conserved hypothetical protein | | Hypothetical proteins-Conserved |
| SP1602 phnA protein | phnA | Transport and binding proteins |
| SP1609 conserved hypothetical protein | | Hypothetical proteins-Conserved |
| SP1621 transcription antiterminator BglG family protein, authentic frameshift | | Transcription |
| SP1625 cadmium resistance transporter, putative | | Transport and binding proteins |
| SP1626 ribosomal protein S15 | rpsO | Protein synthesis |
| SP1642 hypothetical protein | | Hypothetical proteins |
| SP1643 hypothetical protein | | Hypothetical proteins |
| SP1685 conserved hypothetical protein | | Hypothetical proteins-Conserved |
| SP1686 oxidoreductase, Gfo/ldh/MocA family | | Unknown function |
| SP1691 conserved hypothetical protein | | Hypothetical proteins-Conserved |
| SP1701 phospho-2-dehydro-3-deoxyheptonate aldolase | | Amino acid biosynthesis |
| SP1704 ABC transporter, ATP-binding protein | | Transport and binding proteins |
| SP1705 hypothetical protein | | Hypothetical proteins |
| SP1738 guanylate kinase | gmk | Purines, pyrimidines, etc |
| SP1740 conserved hypothetical protein | | Hypothetical proteins-Conserved |
| SP1741 conserved hypothetical protein | | Hypothetical proteins-Conserved |
| SP1743 conserved hypothetical protein | | Hypothetical proteins-Conserved |
| SP1750 conserved hypothetical protein | | Hypothetical proteins-Conserved |
| SP1757 conserved hypothetical protein | | Hypothetical proteins-Conserved |
| SP1762 hypothetical protein | | Hypothetical proteins |
| SP1799 sugar-binding transcriptional regulator, Lacl family | | Regulatory functions |
| SP1801 conserved hypothetical protein | | Hypothetical proteins-Conserved |
| SP1802 hypothetical protein | | Hypothetical proteins |
| SP1812 tryptophan synthase, beta subunit | trpB | Amino acid biosynthesis |
| SP1821 sugar-binding transcriptional regulator, Lacl family | | Regulatory functions |
| SP1831 hypothetical protein | | Hypothetical proteins |
| SP1867 NAD-dependent epimerase/dehydratase family protein | | Energy metabolism |
| SP1892 hypothetical protein | | Hypothetical proteins |
| SP1893 hypothetical protein | | Hypothetical proteins |
| SP1931 hypothetical protein, fusion | | Disrupted reading frame |
| SP1987 ABC transporter, ATP-binding protein | | Transport and binding proteins |
| SP2006 transcriptional regulator ComX1 | comX1 | Regulatory functions |
| SP2030 transketolase | recP | Energy metabolism |
| SP2031 conserved hypothetical protein | | Hypothetical proteins-Conserved |
| SP2036 PTS system, IIA component | | Transport and binding proteins |
| SP2042 ribonuclease P protein component | rnpA | Transcription |
| SP2056 N-acetylglucosamine-6-phosphate deacetylase | nagA | Central intermediary metabolism |
| SP2096 peptidase, M20/M25/M40 family | | Protein fate |
| SP2135 ribosomal protein L33 | rpmG | Protein synthesis |
| SP2186 glycerol kinase | gplK | Energy metabolism |
| SP2187 conserved domain protein | | Hypothetical proteins-Domain |
| SP2206 ribosomal subunit interface protein | yfiA | Protein synthesis |
| SP2209 conserved hypothetical protein | | Hypothetical proteins-Conserved |
| SP2233 hypothetical protein | | Hypothetical proteins |
| SP2234 transcriptional regulator, TetR family | | Regulatory functions |
| SP0651 conserved hypothetical protein | | Hypothetical proteins-Conserved |
| SP0827 conserved hypothetical protein, authentic point mutation | | Disrupted reading frame |
| SP0949 IS1515, transposase, authentic frameshift | | Mobile and extrachromosomal ele |
| SP0952 alanine dehydrogenase, authentic frameshift | ald | Energy metabolism |
| SP1311 IS66 family element, Orf3, degenerate | | Mobile and extrachromosomal ele |
| SP1430 type II restriction endonuclease, putative, authentic point mutation | | DNA metabolism |
| SP1526 ABC transporter, ATP-binding protein authentic frameshift | | Transport and binding proteins |

**Table 2A. Conditionally essential Streptococcus pneumoniae genes identified in blood that encode a predicted surface-localised protein.**

| **SP nr. Annotation** | **Gene** | **Mainrole** |
|---|---|---|
| SP0008 hypothetical protein | | Hypothetical proteins |
| SP0010 conserved domain protein | | Hypothetical proteins-Domain |
| SP0529 transport protein BlpB | blpB | Transport and binding proteins |
| SP0749 branched-chain amino acid ABC transporter, amino acid-binding protein | livj | Transport and binding proteins |
| SP0769 VanZF-related protein | | Unknown function |
| SP0845 lipoprotein | | Cell envelope |
| SP0899 conserved hypothetical protein | | Hypothetical proteins-Conserved |
| SP1232 membrane protein | | Cell envelope |
| SP1437 conserved domain protein | | Hypothetical proteins-Domain |
| SP1690 ABC transporter, substrate-binding protein | | Transport and binding proteins |
| SP1728 hypothetical protein | | Hypothetical proteins |
| SP1796 ABC transporter, substrate-binding protein | | Transport and binding proteins |
| SP1955 hypothetical protein | | Hypothetical proteins |
| SP2010 penicillin-binding protein 2A | pbp2A | Cell envelope |
| SP2051 competence protein CglC | cglC | Cellular processes |
| SP2084 phosphate ABC transporter, phosphate-binding protein | pstS | Transport and binding proteins |
| SP2147 hypothetical protein | | Hypothetical proteins |
| SP2185 hypothetical protein | | Hypothetical proteins |
| SP2197 ABC transporter, substrate-binding protein, putative | | Transport and binding proteins |

**Table 2B. Conditionally essential Streptococcus pneumoniae genes identified in blood that encode a predicted surface-localised protein.**

| **SP nr. Annotation** | **Gene** | **Mainrole** |
|---|---|---|
| SP0062 PTS system, IIC component | | Transport and binding proteins |
| SP0282 PTS system, mannose-specific IID component | | Transport and binding proteins |
| SP0283 PTS system, mannose-specific IIC component | manM | Transport and binding proteins |
| SP0324 PTS system, IIC component | | Transport and binding proteins |
| SP0325 PTS system, IID component | | Transport and binding proteins |
| SP0514 hypothetical protein | | Hypothetical proteins |
| SP1185 PTS system, lactose-specific IIBC components | lace | Transport and binding proteins |
| SP1368 psr protein | | Unknown function |
| SP1684 PTS system, IIBC components | | Transport and binding proteins |
| SP2093 hypothetical protein | | Hypothetical proteins |
| SP2205 DHH subfamily 1 protein | | Unknown function |

**Table 2C. Conditionally essential Streptococcus pneumoniae genes identified in blood that encode a predicted cytoplasm-localised protein.**

| **SP nr. Annotation** | **Gene** | **Mainrole** |
|---|---|---|
| SP0007 S4 domain protein | | Unknown function |
| SP0029 hypothetical protein | | Hypothetical proteins |
| SP0058 transcriptional regulator, GntR family | | Regulatory functions |
| SP0067 hypothetical protein | | Hypothetical proteins |
| SP0091 ABC transporter, permease protein | | Transport and binding proteins |
| SP0097 conserved domain protein | | Hypothetical proteins-Domain |
| SP0099 hypothetical protein | | Hypothetical proteins |
| SP0104 hydrolase, haloacid dehalogenase-like family | | Unknown function |
| SP0108 hypothetical protein | | Hypothetical proteins |
| SP0111 amino acid ABC transporter, ATP-binding protein, putative | | Transport and binding proteins |
| SP0113 argininosuccinate synthase, truncation | argG | Disrupted reading frame |
| SP0116 hypothetical protein | | Hypothetical proteins |
| SP0119 MutT/nudix family protein | | DNA metabolism |
| SP0133 hypothetical protein | | Hypothetical proteins |
| SP0135 glycosyl transferase, putative | | Cell envelope |
| SP0138 hypothetical protein | | Hypothetical proteins |
| SP0139 conserved domain protein | | Hypothetical proteins-Domain |
| SP0176 3,4-dihydroxy-2-butanone 4-phosphate synthase/GTP cyclohydrolase II | ribAB | Biosynthesis of cofactors, etc |
| SP0179 Holliday junction DNA helicase RuvA | ruvA | DNA metabolism |
| SP0180 DNA-3-methyladenine glycosylase I | tag | DNA metabolism |
| SP0206 hypothetical protein | | Hypothetical proteins |
| SP0245 pyruvate formate-lyase-activating enzyme, putative | | Energy metabolism |
| SP0280 ribosomal small subunit pseudouridine synthase A | rsuA-1 | Protein synthesis |
| SP0286 Cof family protein | | Unknown function |
| SP0287 xanthine/uracil permease family protein | | Transport and binding proteins |
| SP0302 conserved domain protein | | Hypothetical proteins-Domain |
| SP0340 autoinducer-2 production protein | luxS | Cellular processes |
| SP0400 trigger factor | tig | Protein fate |
| SP0470 hypothetical protein | | Hypothetical proteins |
| SP0475 hypothetical protein | | Hypothetical proteins |
| SP0488 conserved hypothetical protein | | Hypothetical proteins-Conserved |
| SP0489 PAP2 family protein | | Unknown function |
| SP0507 type I restriction-modification system, S subunit, putative | | DNA metabolism |
| SP0521 HIT family protein | | Unknown function |
| SP0525 blpS protein | blpS | Unknown function |
| SP0540 blpN protein | blpN | Unknown function |
| SP0545 immunity protein BlpY | blpY | Cellular processes |
| SP0550 conserved hypothetical protein | | Hypothetical proteins-Conserved |
| SP0582 hypothetical protein | | Hypothetical proteins |
| SP0603 DNA-binding response regulator VncR | vncR/rr10 | Signal transduction |
| SP0628 HIT family protein | | Unknown function |
| SP0635 hypothetical protein | | Hypothetical proteins |
| SP0678 conserved hypothetical protein | | Hypothetical proteins-Conserved |
| SP0679 hypothetical protein | | Hypothetical proteins |
| SP0687 ABC transporter, ATP-binding protein | | Transport and binding proteins |
| SP0696 hypothetical protein | | Hypothetical proteins |
| SP0698 hypothetical protein | | Hypothetical proteins |
| SP0722 transcriptional activator TenA | tenA | Regulatory functions |
| SP0723 conserved domain protein | | Hypothetical proteins-Domain |
| SP0777 hypothetical protein | | Hypothetical proteins |
| SP0791 oxidoreductase, aldo/keto reductase family | | Unknown function |
| SP0792 hypothetical protein | | Hypothetical proteins |
| SP0793 oxidoreductase, short chain dehydrogenase/reductase family | | Unknown function |
| SP0816 hypothetical protein | | Hypothetical proteins |
| SP0830 hypothetical protein | | Hypothetical proteins |
| SP0831 purine nucleoside phosphorylase, family 2 | deoD | Purines, pyrimidines, etc |
| SP0843 deoxyribose-phosphate aldolase | deoC | Energy metabolism |
| SP0881 thiazole biosynthesis protein Thil | thil | Biosynthesis of cofactors, etc |
| SP0890 integrase/recombinase, phage integrase family | | DNA metabolism |
| SP0893 transcriptional repressor, putative | | Regulatory functions |
| SP0901 hypothetical protein | | Hypothetical proteins |
| SP0925 conserved hypothetical protein | | Hypothetical proteins-Conserved |
| SP0942 IS1381, transposase OrfA | | Mobile and extrachromosomal elemen |
| SP0953 acetyltransferase, GNAT family | | Unknown function |
| SP0954 competence protein CelA | celA | Cellular processes |
| SP0955 competence protein CeIB | celB | Cellular processes |
| SP0958 hypothetical protein | | Hypothetical proteins |
| SP0971 kinase, putative | | Unknown function |
| SP1009 ferrochelatase | hemH | Biosynthesis of cofactors, etc |
| SP1011 GtrA family protein | | Unknown function |
| SP1025 hypothetical protein | | Hypothetical proteins |
| SP1030 conserved hypothetical protein | | Hypothetical proteins-Conserved |
| SP1035 iron-compound ABC transporter, ATP-binding protein | | Transport and binding proteins |
| SP1037 type II restriction endonuclease, putative | | DNA metabolism |
| SP1038 hypothetical protein | | Hypothetical proteins |
| SP1039 hypothetical protein | | Hypothetical proteins |
| SP1050 transcriptional regulator, putative | | Regulatory functions |
| SP1052 phosphoesterase, putative | | Unknown function |
| SP1053 conserved domain protein | | Hypothetical proteins-Domain |
| SP1054 Tn5252, Orf 10 protein | | Mobile and extrachromosomal elemen |
| SP1055 Tn5252, Orf 9 protein | | Mobile and extrachromosomal elemen |
| SP1061 protein kinase, putative | | Regulatory functions |
| SP1072 DNA primase | dnaG | DNA metabolism |
| SP1123 glycogen biosynthesis protein GlgD | glgD | Energy metabolism |
| SP1129 integrase/recombinase, phage integrase family | | DNA metabolism |
| SP1130 transcriptional regulator | | Regulatory functions |
| SP1131 transcriptional regulator, putative | | Regulatory functions |
| SP1137 GTP-binding protein, putative | | Unknown function |
| SP1138 hypothetical protein | | Hypothetical proteins |
| SP1139 hypothetical protein | | Hypothetical proteins |
| SP1149 IS630-Spn1, transposase Orf1 | | Mobile and extrachromosomal elemen |
| SP1166 MATE efflux family protein | | Transport and binding proteins |
| SP1173 conserved hypothetical protein | | Hypothetical proteins-Conserved |
| SP1177 phosphocarrier protein HPr | ptsH | Transport and binding proteins |
| SP1178 NrdH-redoxin | nrdH | Purines, pyrimidines, etc |
| SP1186 PTS system, lactose-specific IIA component | lacF | Transport and binding proteins |
| SP1191 tagatose-6-phosphate kinase | lacC | Energy metabolism |
| SP1192 galactose-6-phosphate isomerase, LacB subunit | lacB | Energy metabolism |
| SP1209 hypothetical protein | | Hypothetical proteins |
| SP1210 hypothetical protein | | Hypothetical proteins |
| SP1215 transporter, FNT family, putative | | Transport and binding proteins |
| SP1233 hypothetical protein | | Hypothetical proteins |
| SP1245 Cof family protein | | Unknown function |
| SP1248 ribonuclease III | mc | Transcription |
| SP1249 conserved hypothetical protein | | Purines, pyrimidines, etc |
| SP1275 carbamoyl-phosphate synthase, large subunit | carB | Purines, pyrimidines, etc |
| SP1282 ABC transporter, ATP-binding protein | | Transport and binding proteins |
| SP1299 ribosomal protein L31 | rpmE | Protein synthesis |
| SP1308 toxin secretion ATP-binding protein, truncation | | Transport and binding proteins |
| SP1314 IS66 family element, Orf1 | | Mobile and extrachromosomal elemen |
| SP1320 v-type sodium ATP synthase, subunit E | ntpE | Transport and binding proteins |
| SP1322 v-type sodium ATP synthase, subunit I | ntpl | Transport and binding proteins |
| SP1323 hypothetical protein | | Hypothetical proteins |
| SP1331 phosphosugar-binding transcriptional regulator, putative | | Regulatory functions |
| SP1332 conserved domain protein | | Hypothetical proteins-Domain |
| SP1333 hypothetical protein | | Hypothetical proteins |
| SP1339 hypothetical protein | | Hypothetical proteins |
| SP1345 hypothetical protein | | Hypothetical proteins |
| SP1349 hypothetical protein | | Hypothetical proteins |
| SP1370 shikimate kinase | aroK | Amino acid biosynthesis |
| SP1384 conserved hypothetical protein | | Hypothetical proteins-Conserved |
| SP1393 conserved hypothetical protein | | Hypothetical proteins-Conserved |
| SP1397 phosphate ABC transporter, ATP-binding protein, putative | | Transport and binding proteins |
| SP1406 hypothetical protein | | Hypothetical proteins |
| SP1422 hypothetical protein | | Hypothetical proteins |
| SP1423 transcriptional repressor, putative | | Regulatory functions |
| SP1426 ABC transporter, ATP-binding protein | | Transport and binding proteins |
| SP1429 peptidase, U32 family | | Protein fate |
| SP1442 IS66 family element, Orf2 | | Mobile and extrachromosomal elemen |
| SP1450 platelet activating factor, putative | | Fatty acid and phospholipid metab |
| SP1465 hypothetical protein | | Hypothetical proteins |
| SP1467 conserved hypothetical protein | | Hypothetical proteins-Conserved |
| SP1478 oxidoreductase, aldo/keto reductase family | | Unknown function |
| SP1495 hypothetical protein | | Hypothetical proteins |
| SP1502 amino acid ABC transporter, permease protein | | Transport and binding proteins |
| SP1512 ATP synthase F0, B subunit | atpF | Energy metabolism |
| SP1537 general stress protein 13, putative | | Cellular processes |
| SP1547 conserved hypothetical protein | | Hypothetical proteins-Conserved |
| SP1552 cation efflux family protein | | Transport and binding proteins |
| SP1563 pyridine nucleotide-disulphide oxidoreductase family protein | | Unknown function |
| SP1599 tRNA pseudouridine synthase A | truA | Protein synthesis |
| SP1600 hypothetical protein | | Cell envelope |
| SP1602 phnA protein | phnA | Transport and binding proteins |
| SP1616 ribulose-phosphate 3-epimerase family protein | | Energy metabolism |
| SP1619 PTS system, IIA component | | Transport and binding proteins |
| SP1620 PTS system, nitrogen regulatory component IIA, putative | | Transport and binding proteins |
| SP1630 hypothetical protein | | Hypothetical proteins |
| SP1631 threonyl-tRNA synthetase | thrS | Protein synthesis |
| SP1639 IS1167, transposase | | Mobile and extrachromosomal elemen |
| SP1642 hypothetical protein | | Hypothetical proteins |
| SP1643 hypothetical protein | | Hypothetical proteins |
| SP1647 endopeptidase O | pepO | Protein fate |
| SP1695 acetyl xylan esterase, putative | | Energy metabolism |
| SP1718 hypothetical protein | | Hypothetical proteins |
| SP1759 preprotein translocase, SecA subunit | secA | Protein fate |
| SP1781 conserved hypothetical protein | | Hypothetical proteins-Conserved |
| SP1787 hypothetical protein | | Hypothetical proteins |
| SP1789 hypothetical protein | | Hypothetical proteins |
| SP1794 hypothetical protein | | Hypothetical proteins |
| SP1799 sugar-binding transcriptional regulator, Lacl family | | Regulatory functions |
| SP1801 conserved hypothetical protein | | Hypothetical proteins-Conserved |
| SP1808 type IV prepilin peptidase, putative | | Protein fate |
| SP1810 hypothetical protein | | Hypothetical proteins |
| SP1811 tryptophan synthase, alpha subunit | trpA | Amino acid biosynthesis |
| SP1822 conserved domain protein | | Hypothetical proteins-Domain |
| SP1823 MgtC/SapB family protein | | Transport and binding proteins |
| SP1824 ABC transporter, permease protein | | Transport and binding proteins |
| SP1825 ABC transporter, ATP-binding protein | | Transport and binding proteins |
| SP1849 DpnD protein | dpnD | Unknown function |
| SP1851 conserved hypothetical protein | | Hypothetical proteins-Conserved |
| SP1863 transcriptional regulator, MarR family | | Regulatory functions |
| SP1864 conserved hypothetical protein | | Hypothetical proteins-Conserved |
| SP1894 sucrose phosphorylase | gftA | Energy metabolism |
| SP1899 msm operon regulatory protein | msmR | Regulatory functions |
| SP1903 conserved hypothetical protein | | Hypothetical proteins-Conserved |
| SP1931 hypothetical protein, fusion | | Disrupted reading frame |
| SP1950 bacteriocin formation protein, putative | | Cellular processes |
| SP1963 CBS domain protein | | Unknown function |
| SP1979 pur operon repressor | purR | Regulatory functions |
| SP1987 ABC transporter, ATP-binding protein | | Transport and binding proteins |
| SP1988 immunity protein, putative | | Cellular processes |
| SP1989 transcriptional regulator PlcR, putative | | Regulatory functions |
| SP2016 nicotinate-nucleotide pyrophosphorylase | nadC | Biosynthesis of cofactors, etc |
| SP2021 glycosyl hydrolase, family 1 | | Energy metabolism |
| SP2023 PTS system, IIB component | | Transport and binding proteins |
| SP2028 phosphotyrosine protein phosphatase | | Regulatory functions |
| SP2031 conserved hypothetical protein | | Hypothetical proteins-Conserved |
| SP2054 conserved hypothetical protein | | Hypothetical proteins-Conserved |
| SP2064 hydrolase, haloacid dehalogenase-like family | | Unknown function |
| SP2087 phosphate ABC transporter, ATP-binding protein | pstB | Transport and binding proteins |
| SP2088 phosphate transport system regulatory protein PhoU | phoU | Regulatory functions |
| SP2089 transposase, IS1380-Spn1 related, truncation | | Mobile and extrachromosomal elemen |
| SP2090 transcriptional regulator | | Regulatory functions |
| SP2096 peptidase, M20/M25/M40 family | | Protein fate |
| SP2111 malA protein | | Energy metabolism |
| SP2115 hypothetical protein | | Hypothetical proteins |
| SP2122 conserved hypothetical protein | | Hypothetical proteins-Conserved |
| SP2135 ribosomal protein L33 | rpmG | Protein synthesis |
| SP2139 hypothetical protein | | Hypothetical proteins |
| SP2148 arginine deiminase | arcA | Energy metabolism |
| SP2150 ornithine carbamoyltransferase | argF | Energy metabolism |
| SP2151 carbamate kinase | arcC | Energy metabolism |
| SP2157 alcohol dehydrogenase, iron-containing | | Energy metabolism |
| SP2168 fucose operon repressor, putative | | Regulatory functions |
| SP2206 ribosomal subunit interface protein | yfiA | Protein synthesis |
| SP2229 tryptophanyl-tRNA synthetase | trps | Protein synthesis |
| SP2233 hypothetical protein | | Hypothetical proteins |
| SP2238 conserved hypothetical protein | | Hypothetical proteins-Conserved |
| SP2240 spspoJ protein | | Cellular processes |
| SP0949 IS1515, transposase, authentic frameshift | | Mobile and extrachromosomal elemen |
| SP2046 conserved domain protein, authentic frameshift | | Disrupted reading frame |
| SP2123 transcriptional regulator, authentic frameshift | | Regulatory functions |
| SP2232 conserved hypothetical protein, authentic frameshift | | Disrupted reading frame |

**Table 3. Genes that are common to Table 1 and 2.**

| Table 1A/2A | Table 1B/2B | Table 1C/2C |
|---|---|---|
| | | SP0058 |
| | | SP0113 |
| | | SP0116 |
| | | SP0206 |
| | SP0282 | SP0245 |
| | SP0283 | SP0280 |
| | | SP0302 |
| | | SP0340 |
| | | SP0488 |
| | | SP0489 |
| | SP0514 | SP0521 |
| | | SP0525 |
| | | SP0687 |
| SP0749 | | SP0696 |
| SP0769 | | SP0777 |
| | | SP0816 |
| | | SP0830 |
| | | SP0831 |
| | | SP0881 |
| | | SP0893 |
| | | SP1011 |
| | | SP1025 |
| | | SP1039 |
| | | SP1052 |
| | | SP1053 |
| | | SP1054 |
| | | SP1055 |
| | | SP1061 |
| | | SP1177 |
| | | SP1245 |
| | | SP1299 |
| | | SP1320 |
| | | SP1333 |
| | SP1368 | SP1370 |
| | | SP1393 |
| | | SP1397 |
| | | SP1422 |
| | | SP1429 |
| SP1437 | | SP1465 |
| | | SP1467 |
| | | SP1478 |
| | | SP1537 |
| | | SP1563 |
| | | SP1602 |
| | | SP1642 |
| | | SP1643 |
| SP1690 | | SP1799 |
| | | SP1801 |
| | | SP1931 |
| SP1955 | | SP1987 |
| SP2010 | | SP2031 |
| SP2084 | | SP2096 |
| SP2147 | | SP2135 |
| SP2197 | SP2205 | SP2206 |
| | | SP2233 |

Table 4. Genes found in the GAF screen, that have been identified in literature as potential vaccine candidates. Indicated literature references are: H: Hava et al., 2002, Mol. Microbiol. 45:1389-1406; L: Lau et al., 2001, Mol. Microbiol. 40:555-571; P: Polissi et al., 1998, Infect. Immun. 66:5620-5629; 1: Adamou et al., 2001, Infect. Immun. 69:949-958; 2: Berry et al., 1996, J. Bacteriol. 178:4854-4860; 3: Spellerberg et al., 1996, Mol. Microbiol. 19:803-813; **4:** Garcia et al., 1999, Mol. Microbiol. 31:1275-1281; **5:** Gosink et al., 2000, Infect. immun. 68:5690-5695; **6:** Kwon et al., 2003, Infect. Immun. 71:3757-3765; **7:** Orihuela et al., 2004, Infect. Immun. 72:5582-5596; **8:** Throup et al., 2000, Mol. Microbiol. 35:566-576; **9:** Tong et al., 2000, Infect. Immun. 68:921-924; **10:** Vollmer et al., 2002, Infect. Immun. 70:7176-7178; **11:** Caimano, M.J. et al., in: Streptococcus pneumoniae - Molecular biology and mechanisms of disease, A. Tomasz (Ed.), Mary Ann Liebert, Larchmont, NY, 2000, p.115.

| Locus. Annotation | Ref |
|---|---|
| SP0044 phosphoribosylaminoimidazole-succinocarboxamide synthase | P |
| SP0049 vanZ protein, putative | H |
| SP0050 phosphoribosylaminoimidazolecarboxamide formyltransferase/IMP cyclohydrolase | H |
| SP0054 phosphoribosylaminoimidazole carboxylase, ATPase subunit | P |
| SP0083 DNA-binding response regulator | 8 |
| SP0100 conserved hypothetical protein | H |
| SP0143 conserved domain protein | P |
| SP0146 conserved hypothetical protein | H |
| SP0157 hypothetical protein | H |
| SP0177 riboflavin synthase, alpha subunit | H |
| SP0198 hypothetical protein | H |
| SP0199 cardiolipin synthetase | H |
| SP0246 transcriptional regulator, DeoR family | H |
| SP0247 transcriptional regulator | H |
| SP0251 formate acetyltransferase, putative | L,H |
| SP0253 glycerol dehydrogenase | H |
| SP0268 alkaline amylopullulanase, putative | H |
| SP0320 oxidoreductase, short chain dehydrogenase/reductase family | H |
| SP0332 hypothetical protein | H |
| SP0350 capsular polysaccharide biosynthesis protein Cps4E | 11 |
| SP0351 capsular polysaccharide biosynthesis protein Cps4F | 11 |
| SP0358 capsular polysaccharide biosynthesis protein cps4J | 11 |
| SP0390 choline binding protein G | 5 |
| SP0396 PTS system, mannitol-specific 11A component | H |
| SP0454 hypothetical protein | H |
| SP0467 sortase, putative | H |
| SP0510 type I restriction-modification system, R subunit | H |
| SP0527 putative histidine kinase BlpH | 8 |
| SP0530 BlpC ABC transporter, ATP-binding protein, authentic frameshift | H |
| SP0586 5,10-methylenetetrahydrofolate reductase, putative | H |
| SP0609 amino acid ABC transporter, AA-binding protein | P |
| SP0633 hypothetical protein | H |
| SP0665 chorismate binding enzyme | H |
| SP0728 hypothetical protein | H |
| SP0729 cation-transporting ATPase, E1-E2 family | H |
| SP0730 pyruvate oxidase | 3 |
| SP0742 conserved hypothetical protein | P |
| SP0746 ATP-dependent Clp protease, proteolytic subunit | 6 |
| SP0785 conserved hypothetical protein | H |
| SP0789 conserved hypothetical protein | H |
| SP0820 ATP-dependent Clp protease, ATP-binding subunit ClpE | P |
| SP0829 phosphopentomutase | H |
| SP0886 type I restriction-modification system, M subunit, putative | H |
| SP0892 type I restriction-modification system, R subunit, putative | H |
| SP0943 Gid protein | H |
| SP0948 PhoH family protein | P |
| SP0965 endo-beta-N-acetylglucosaminidase | 4 |
| SP1004 conserved hypothetical protein | 1 |
| SP1029 RNA methyltransferase, TrmA family | H |
| SP1033 iron-compound ABC transporter, permease protein | L |
| SP1045 conserved hypothetical protein TIGR00147 | H |
| SP1112 degV family protein | H |
| SP1115 transcriptional regulator MutR, putative | H |
| SP1121 1,4-alpha-glucan branching enzyme | H |
| SP1127 hypothetical protein | H |
| SP1193 galactose-6-phosphate isomerase, LacA subunit | H |
| SP1326 neuraminidase, putative | 2 |
| SP1328 sodium:solute symporter family protein | H |
| SP1342 toxin secretion ABC transporter, ATP-binding/permease protein | P |
| SP1343 prolyl oligopeptidase family protein | H |
| SP1344 conserved hypothetical protein | H |
| SP1389 spermidine/putrescine ABC transporter, ATP-binding protein | P |
| SP1396 phosphate ABC transporter, ATP-binding protein, putative | H |
| SP1434 ABC transporter, ATP-binding/permease protein | H |
| SP1469 NADH oxidase | L |
| SP1479 peptidoglycan N-acetylglucosamine deacetylase A | 10 |
| SP1527 oligopeptide ABC transporter, oligopeptide-binding protein AliB | L |
| SP1544 aspartate aminotransferase | H |
| SP1623 cation-transporting ATPase, E1-E2 family | P |
| SP1645 GTP pyrophosphokinase | H |
| SP1693 neuraminidase A, authentic frameshift | 9 |
| SP1706 hypothetical protein | H |
| SP1717 ABC transporter, ATP-binding protein | H |
| SP1753 sodium/dicarboxylate symporter family protein | 7 |
| SP1771 glycosyl transferase, family 2/glycosyl transferase family 8 | H |
| SP1782 ribosomal protein L11 methyltransferase | P |
| SP1800 transcriptional activator, putative | H |
| SP1830 phosphate transport system regulatory protein PhoU, putative | H |
| SP1847 xanthine phosphoribosyltransferase | H |
| SP1939 MATE efflux family protein DinF | H |
| SP2052 competence protein CgIB | H |
| SP2082 response regulator | 8 |
| SP2083 sensor histidine kinase PnpS | 8 |
| SP2091 glycerol-3-phosphate dehydrogenase (NAD(P)+) | L |
| SP2098 membrane protein | H |
| SP2116 conserved domain protein | P |
| SP2145 antigen, cell wall surface anchor family | L,H |
| SP2176 D-alanine-activating enzyme | H |
| SP2175 dltB protein | H |
| SP2231 ABC transporter, permease protein, putative | H |

The proteins encoded by the genes listed in Table 1A-C and/or 2A-C may be used to produce vaccines or antibodies of the invention. A suitable source of such proteins is for instance *Streptococcus pneumoniae.* The protein may be used non-purified (associated with in intact cells), partially purified (associated with membrane fragments or other cellular constituents), or purified (i.e. isolated and essentially free of other cellular constituents). Having prepared purified or partially purified one or more of the proteins it is possible to prepare a substantially pure preparation of such a protein. Although numerous methods and strategies for protein purification are known in the art it will be most convenient to purify such a protein by either electrophoresis using for instance a sodium dodecylsulphate-polyacrylamide gel (SDS-PAGE) or by affinity chromatography. Each of these methods will be described below.

A protein encoded by a gene listed in Table 1A-C and/or 2A-C may be separated from other proteins by electrophoresis using for instance Tricine-SDS-PAGE (Schagger and Von Jagow (1987) Analytical Biochemistry 166, 368-379) or Glycine-SDS-PAGE (Laemmli (1970) Nature 227, 680-685). Other electrophoresis systems that are capable of resolving the various proteins comprised in a bacterial lysate, or transcribed from its genome and expressed in a suitable expression system, may of course also be employed, such as non-denaturing gel electrophoresis. The area of the PAGE gel including the target protein may be excised and the target polypeptides may be eluted therefrom. The protein of interest may be identified by its mobility relative to reference polypeptides in a gel. To increase purity the eluted protein may be run on a second SDS-PAGE gel and eluted a second time. The protein or peptide contained in the excised gel fragment may then be eluted again and is suitable for use in immunization or in protein sequencing.

The protein may also be purified by affinity chromatography using an antibody (such as a monoclonal antibody) that specifically binds to said protein. The antibody may be covalently coupled to solid supports such as celluloses, polystyrene, polyacrylamide, cross-linked dextran, beaded agarose or controlled pore glass using bifunctional coupling agents that react with functional groups on the support and functional groups (i.e., reactive amino acid side chains) on the antibody molecule. Such methods are readily available to the skilled person. The resulting antibody-bearing solid phase is contacted with purified or partially purified protein under reducing conditions using pH, ionic strength, temperature and residence times that permit the protein to bind to the immobilized antibody. The protein is eluted from the column by passing an eluent that dissociates hydrogen bonds through the bed. Buffers at specific pH or NaCl solutions above about 2 M are commonly used eluents.

Methods for carrying out affinity chromatography using antibodies as well as other methods for immunoaffinity purification of proteins are well known in the art (see e.g., Harlow and Lane, (1988) Antibodies: A Laboratory Manual. Cold Spring Harbor Laboratory, Cold Spring Harbor, NY).

With the teachings provided herein, the skilled person is capable of isolating a protein encoded by a gene listed in Table 1 and/or 2 and test it for its immunogenic properties, e.g. by performing an opsonophagocytosis assay as described in WO 01/12219.

### Antibody production

Antibodies, either monoclonal or polyclonal, can be generated to a purified or partially purified protein or peptide fragment encoded by a gene listed in Table 1 and/or 2 in a variety of ways known to those skilled in the art including injection of the protein as an antigen in animals, by hybridoma fusion, and by recombinant methods involving bacteria or phage systems (see Harlow and Lane (1988) *supra.;* Marks et al., (1992) Journal of Biological Chemistry, 267, 16007-16010; Marks et al., (1992) Biotechnology 10: 779:783; Lowman et al., (1991) Biochem. 30(45): 10832-8; Lerner et al., (1992) Science 258:1313-1314, each of which references discloses suitable methods).

Antibodies against a protein encoded by a gene listed in Table 1 and/or Table 2 or functional homologues thereof, may be produced by immunizing an appropriate vertebrate, preferably mammalian host, *e.g.,* rabbits, goats, rats and mice or chicken with the protein alone or in conjunction with an adjuvant. Usually two or more immunizations will be involved, and the blood or spleen will be harvested a few days after the last injection. For polyclonal antisera, the immunoglobulins may be precipitated, isolated and (affinity) purified. For monoclonal antibodies, the splenocytes will normally be fused with an immortalized lymphocyte, e.g., a myeloid line, under selective conditions for hybridomas. The hybridomas may then be cloned under limiting dilution conditions and their supernatants screened for antibodies having the desired specificity. Techniques for producing (monoclonal) antibodies and methods for their preparation and use in various procedures are well known in the literature (see *e.g.* U.S. Pat. Nos. 4,381,292, 4,451,570, and 4,618,577; Harlow, E. and Lane, D. (1988) Antibodies: A Laboratory Manual. Cold Spring Harbor Laboratory, Cold Spring Harbor, NY.; Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons; Rose, N., DeMacrio, E., Fahey, J., Friedman, H., Penn, G. (1997) Manual of Clinical Laboratory Immunology. American Soc. Microbiology Press, Washington, D.C Coligan, J.E., Kruisbeek, A.M., Margulies, D.H., Shevach, E.M. Strober, W. (Eds.) (1997) Current Protocols in Immunology. John Wiley & Sons Inc. Baltimore). Typically, an antibody directed against a protein will have a binding affinity of at least 1x10⁵-1x10⁷ M⁻¹.

A recombinant protein or functional homologues thereof, such as may be obtained by expressing a gene from Table 1 and/or 2 in a suitable expression system, is preferred as the antigen in methods for producing an antibody. However, purified proteins may also be used, as well as protein fragments. Antigens suitable for antibody production include any fragment of a protein that elicits an immune response in a mammal exposed to said protein. Preferred antigens of the invention include those fragments that comprise the antigenic determinants, although any region of the proteins encoded by the genes of Tables 1 and/or 2 may in principle be used.

Methods for cloning genomic sequences such as the genes listed in Table 1 and/or 2, for manipulating the genomic sequences to and from expression vectors, and for expressing the protein encoded by the genomic sequence in a heterologous host are well-known, and these techniques can be used to provide the expression vectors, host cells, and the cloned genomic sequences encoding the protein, functional homologues or fragments thereof, which sequences are to be expressed in a host to produce antibodies for use in methods of the present invention (see for instance Sambrook, J., Russell D.W., Sambrook, J. (2001) Molecular Cloning: a Laboratory Manual. Cold Spring Harbor Laboratory Press, Plainview, N.Y., and Ausubel, *et al., supra).*

A variety of expression systems may be used to produce antigens for use in methods of the present invention. For instance; a variety of expression vectors suitable to produce proteins in *Escherichia coli, Lactococcus lactis, Bacillus subtilis,* yeast, insect cells, plant cells and mammalian cells have been described, any of which might be used to produce an antigen suitable to be included in a vaccine or useful to produce an antibody or fragment thereof. Of course *Streptococcus pneumoniae* itself may also be used as an expression vector for this purpose.

One use of antibodies of the invention is to provide active ingredients for a pharmaceutical composition capable of inhibiting virulence or growth of a *Streptococcus pneumoniae* pathogen. Another use of antibodies of the invention is to screen cDNA expression libraries for identifying clones containing cDNA inserts that encode proteins of interest or structurally-related, immuno-cross-reactive proteins. Such screening of cDNA expression libraries is well known in the art (see e.g. Young R.A., Davis, R.W. (1983) Proc. Natl. Acad. Sci. U.S.A. 80:1194-1198), to which reference is made in this context, as well as other published sources. Another use of these antibodies is for use in affinity chromatography for purification of the protein to which it has been elicited or functional homologues thereof. These antibodies are also useful for assaying for infection with *Streptococcus pneumoniae.*

### Antigen Epitopes

The antigen epitopes of this invention, which alone or together form an antigenic determinant of *Streptococcus pneumoniae,* are molecules that are immunoreactive with monoclonal antibodies and whose binding to an antigen of the bacterial pathogen cell prevents the virulence and/or growth of said cell. Systematic techniques for identifying these epitopes are known in the art, as described in US 4,708,871, which is incorporated herein by reference. Typically, these epitopes are short amino acid sequences. These sequences may be embedded in the sequence of longer peptides or proteins, as long as they are accessible.

The epitopes of the invention may be prepared by standard peptide synthesis techniques, such as solid-phase synthesis. Alternatively, the sequences of the invention may be incorporated into larger peptides or proteins by recombinant methods. This is most easily accomplished by preparing a DNA cassette which encodes the sequence of interest, and ligating the cassette into DNA encoding the protein to be modified at the appropriate site. The sequence DNA may be synthesized by standard synthetic techniques, or may be excised from the phage pIII gene using the appropriate restriction enzymes. Epitopes identified herein may be prepared by simple solid-phase techniques. The minimum binding sequence may be determined systematically for each epitope by standard methods, for example, employing the method described in US 4,708,871. Briefly, one may synthesize a set of overlapping oligopeptides derived from an antigen bound to a solid phase array of pins, with a unique oligopeptide on each pin. The pins are arranged to match the format of a 96-well microtiter plate, permitting one to assay all pins simultaneously, e.g., for binding to a monoclonal antibody. Using this method, one may readily determine the binding affinity for every possible subset of consecutive amino acids.

### Antibody Formulations and Methods of Administration

The antibodies of this invention are administered at a concentration that is therapeutically effective to prevent or treat infections by *Streptococcus pneumoniae.* To accomplish this goal, the antibodies may be formulated using a variety of acceptable excipients known in the art. Typically, the antibodies are administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Before administration to patients, formulants (components other than the active ingredient in a product that can have many functions, such as carrier and excipients) may be added to the antibodies. A liquid formulation is preferred. For example, these formulants may include oils, polymers, vitamins, carbohydrates, amino acids, salts, buffers, albumin, surfactants, or bulking agents.

Additionally, antibodies can be chemically modified by covalent conjugation to a polymer to increase their circulating half-life, for example. Preferred polymers are polyethylene glycol (PEG) and polyoxyethylated polyols, such as polyoxyethylated sorbitol, polyoxyethylated glucose, polyoxyethylated glycerol (POG). The PEG has a preferred average molecular weight between 1,000 and 40,000, more preferably between 2,000 and 20,000, most preferably between 3,000 and 12,000.

Another drug delivery system for increasing circulatory half-life is the liposome. Methods of preparing liposome delivery systems are discussed in Gabizon et al., Cancer Research (1982) 42:4734; Cafiso, Biochem. Biophys. Acta (1981) 649:129; and Szoka, Ann. Rev. Biophys. Eng. (1980) 9:467. Other drug delivery systems are known in the art and are described in e.g., Poznansky et al., Drug Delivery Systems (R. L. Juliano, Ed., Oxford, N.Y. 1980), pp. 253-315; M. L. Poznansky, Pharm. Revs. (1984) 36:277.

After a liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is preferably administered to subjects using those methods that are known to those skilled in the art.

As stated above, the antibodies and compositions of this invention are used to treat human patients to prevent or treat *Streptococcus pneumoniae* infections. The preferred route of administration is parenterally. In parenteral administration, the compositions of this invention will be formulated in a unit dosage injectable form such as a solution, suspension or emulsion, in association with a pharmaceutically acceptable parenteral vehicle. Such vehicles are inherently nontoxic and nontherapeutic. Examples of such vehicles are saline, Ringer's solution, dextrose solution, and Hanks' solution. Nonaqueous vehicles such as fixed oils and ethyl oleate may also be used. A preferred vehicle is 5% dextrose in saline. The vehicle may contain minor amounts of additives such as substances that enhance isotonicity and chemical stability, including buffers and preservatives. However, also administration routes other than parenteral (e.g. oral, intranasal, rectal, see hereinbelow with regard to vaccine formulations of the invention) can be applicable for certain embodiments of the invention.

The dosage and mode of administration will depend on the individual. Generally, the compositions are administered so that antibodies are given at a dose between 1 µg/kg and 20 mg/kg, more preferably between 20 µg/kg and 10 mg/kg, most preferably between 1 and 7 mg/kg. Preferably, it is given as a bolus dose, to increase circulating levels by 10-20 fold and for 4-6 hours after the bolus dose. Continuous infusion may also be used after the bolus dose. If so, the antibodies may be infused at a dose between 5 and 20 µg/kg/minute, more preferably between 7 and 15 µg/kg/minute.

The antibody of the present invention may be used prior to infection as a precaution, or after infection has occurred as a therapeutic treatment. Preferably, the therapeutic use of the antibodies as described herein or fragments thereof include administration prior or during the acute invasive phase of the disease.

### Vaccine Formulations and Methods of Administration

The vaccine antigens of this invention are administered at a concentration that is therapeutically effective to prevent or treat infections by *Streptoccus pneumoniae.* To accomplish this goal, the vaccines may be formulated using a variety of acceptable excipients known in the art. Typically, the vaccines are administered by injection, either intravenously or intraperitoneally. Methods to accomplish this administration are known to those of ordinary skill in the art.

Preferably the vaccine contains at least 50 µg of antigenic mass per dose, and most preferably 80 µg per dose. The antigenic mass being the mass of the antigen protein. Vaccines according to the present invention with an antigenic mass up to 275 µg per dose could even be prepared, and such vaccines may still not elicit local reactions at the injection site. Of course even more micrograms of antigen can be put in a vaccine dose of a vaccine according to the invention, but if the protection obtained with the vaccine is not improved with a higher dose the increase in antigenic load only results in the vaccine being more expensive than necessary. In addition an increasing dose of antigen may eventually lead to unacceptable local reactions at the injection site, which should be avoided.

A vaccine according to the invention may contain a (partially) purified or recombinant protein encoded by a gene listed in Tables 1 and/or 2 or an antigenic part thereof, wherein said recombinant protein is preferably produced by way of expression from a expression vector in suitable host cells, said expression vector containing the gene sequence or an immunogenic part thereof under control of a suitable promoter. Several suitable expression systems are known in the art and may be used in a method to prepare a vaccine according to the invention.

A vaccine according to the invention may further comprise a suitable adjuvant. Many adjuvant systems are known in the art, for example commonly used oil in water adjuvant systems. Any suitable oil may be used, for example a mineral oil known in the art for use in adjuvantia. The oil phase may also contain a suitable mixture of different oils, either mineral or non-mineral. Suitable adjuvantia may also comprise vitamin E, optionally mixed with one or more oils. The water phase of an oil in water adjuvanted vaccine will contain the antigenic material. Suitable formulations will usually comprise from about 25-60% oil phase (40-75% water phase). Examples of suitable formulations may comprise 30% water phase and 70% oil phase or 50% of each. Especially preferred is a non-recombinant lactococcal-based vaccine displaying pneumococcal antigens. The lactococcal-derived bacterial shaped particles are non-living and are designated Gram-positive Enhancer Matrix (GEM) particles (Van Roosmalen, M.L. et al., 2006, Methods 38:144-149). These GEM particles are deprived of surface proteins and the intracellular content is largely degraded (Bosma, T. et al., 2006, Appl. Environ. Microbiol. 72:880-889). The GEM particles can be used as anchoring and delivery vehicle for pneumococcal proteins (see Audouy, S.A.L. et al., 2007, Vaccine 25(13):2497).

The vaccine formulations of the present invention may be used in prophylactic methods of the invention by immunizing a subject by introducing said formulations into said subject subcutaneously, intramuscularly, intranasally, intradermally, intravenously, transdermally, transmucosally, orally, or directly into a lymph node. In another embodiment, the composition may be applied locally, near a local pathogen reservoir against which one would like to vaccinate.

The present invention further provides a method for the manufacture of a vaccine intended for the protection of a subject against pneumococcal infection, wherein said vaccine is combined with a pharmaceutically acceptable diluent, carrier, excipient or adjuvant therefore, such that a formulation is provided which can provide a dose of at least 20 µg protein in a single administration event.

A vaccine (prepared by a method) according to the invention can be used in a method to protect a subject against pneumococcal infection.

To provide adequate protection the vaccine is preferably administered in a 2 shot vaccination regimen, whereby the first shot (priming vaccination) and second shot (boosting vaccination) are given to the subject with a interval of about 3 weeks. In this way the subject will have obtained full protection against pneumococcal infection. The vaccination is very favourable for young children.

A vaccine according to the invention can comprise more than one antigen capable of eliciting antibodies against *Streptococcus pneumoniae.* These antigens can be chosen from the proteins encoded by the genes listed in Table 1 and/or 2, or aditionally known antigens, such as those listed in the introduction above may be added.

Further, the genes of Table 1 and/or 2 and/or the proteins encoded by said genes provide excellent targets for small chemical molecules. For finding novel antibiotic compounds a screen with any of these genes and proteins would be suitable.

### EXAMPLES

### A. MOUSE INFECTION MODELS

Nine-week old, female outbred CD-1 mice (Harlan, Horst, the Netherlands) were used for all infection models. Aliquots of bacteria stored at -80°C were rapidly thawed, harvested by centrifugation, and resuspended in sterile PBS to give the required amount of CFU/ml. For the pneumonia model, mice were lightly anaesthetized with 2.5% (v/v) isoflurane/O₂, and infected intranasally by pipetting 50 µl of inoculum onto the nostrils of mice held in upright position. At predetermined times after infection, groups of mice were sacrificed by injection anaesthesia, and blood samples were removed by cardiac puncture using a 1 ml syringe. Bacteria were recovered from the nasopharynx by flushing the nostrils with 2 ml sterile PBS (nasopharyngeal lavage, NPL). Bronchoalveolar lung lavage (BAL) was performed by flushing the lungs with 2 ml sterile PBS, after which lungs were removed from the body and homogenized in 2 ml of sterile PBS using a hand held homogeniser. In the colonization model, mice were infected intranasally with 10 µl of inoculum, a volume small enough to only infect the nose (nasopharynx) of the mice. At predetermined time-points after infection, NPL was collected as described above. In the bacteraemia model, mice were infected in a tail vein with a 100 µl inoculum. Bacteria were recovered from the blood by a lateral tail vein puncture from the same mouse at 0, 12 and 24 hours post-infection. The number of viable bacteria in NPL, BAL, blood and homogenized lungs was determined by plating on serial dilutions on agar plates. All animal experiments were performed with approval of the Radboud University Nijmegen Medical Centre Commitee for Animal Ethics.

### B. GENOMIC ARRAY FOOTPRINTING

***DNA isolation.*** Chromosomal DNA was isolated from pneumococcal cultures by cetyl-trimethylammonium bromide (CTAB) extraction using standard protocols.

***Generation of transposon mutant libraries.*** For *in vitro* transposon mutagenesis, 1 µg of pneumococcal DNA was incubated in the presence of purified HimarC9 transposase with 0.5 µg of plasmid pR412T7 (Bijlsma, J.J.E. et al., 2007, Appl. Environm. Microbiol. 73(5):1514-1524) as donor for *mariner* transposon conferring spectinomycin resistance, respectively. After repair of the resulting transposition products with T4 DNA polymerase and *Escherichia coli* DNA ligase, the DNA was used for transformation of strain TIGR4). Preparation and transformation of precompetent *Streptococcus pneumoniae* cell stocks was performed essentially as described. Briefly, cCAT medium was inoculated with several colonies and grown to an optical density at 620 nm (OD₆₂₀) of 0.25-0.3. After a 30-fold dilution of the culture in CTM medium, cells were grown to an OD₆₂₀ of 0.1, pelleted, resuspended in 0.1 volume of CTM-pH7.8 (CTM adjusted to pH 7.8 with NaOH) containing 15% glycerol, and stored at -80°C. For transformation, precompetent TIGR4 cells were grown for 15 minutes at 37°C in a 10-fold volume of CTM-pH7.8 supplemented with 100 ng/ml CSP-2. After addition of DNA, cultures were incubated for 30 min at 32°C, followed by a two-hour incubation at 37°C. After overnight growth on selective plates containing 150 µg/ml spectinomycin, the required number of colonies were scraped from the plates, pooled, grown to mid-log phase in 20 ml of GM17 medium supplemented with spectinomycin, and stored at -80°C.

### Probe generation, labeling, and microarray hybridization.

Chromosomal DNA from challenged and non-challenged mutant libraries was digested with *Alu*I endonuclease. The resulting DNA fragments were purified using Qiagen MinElute columns and used as a template for an *in vitro* T7 RNA polymerase reaction using the Ambion T7 MegaScript kit. After removal of template DNA by DNAseI treatment, RNA was purified using Qiagen RNeasy MinElute columns. Fluorescent Cy3/Cy5-labeled dUTP nucleotides were incorporated by reverse transcription using Superscript III. Labeled challenged cDNA was mixed with labeled non-challenged cDNA and purified by washing and ultrafiltration using GFX and Microcon-30 spin columns. Samples were suspended in Slidehyb buffer 1 and hybridized in to pneumococcal microarrays for 16 hours at 45°C. Microarrays used in this study were constructed as described and contain amplicons representing 2,087 ORFs of *Streptococcus pneumoniae* TIGR4 and 184 ORFs unique for *Streptococcus pneumoniae* R6, all spotted in duplicate. After hybridization, microarrays were washed with 2xSSC, 0.25% SDS for 5 min, followed by 2 washes in 1x SSC and 0.5x SSC for 5 min each. Finally, slides were dipped into H₂O and dried by centrifugation for 5 min at 50 x g.

***Microarray data analysis.*** Dual channel array images were acquired on a GenePix 4200AL microarray scanner and analyzed with GenePix Pro software. Spots were screened visually to identify those of low quality and removed from the data set prior to analysis. A net mean intensity filter based on hybridization signals obtained with amplicons representing open reading frames unique for *Streptococcus pneumoniae* strain R6 was applied in all experiments. Slide data were processed and normalized using MicroPreP. Further analysis was performed using a Cyber-T implementation of the Student's t test. This web-based program lists the ratios of all intra-replicates (duplicate spots) and inter-replicates (different slides), the mean ratios per gene, and standard deviations and (Bayesian) *p*-values assigned to the mean ratios. For identification of conditionally essential genes, only genes with a minimum of five reliable measurements, a Bayesian p-value < 0.001, a false-discovery rate (FDR) < 0.05, and a standard deviation < 0.85 x abs (ratio) were included. Furthermore, an average fold-change cut-off of 2.5 was applied.

***In silico analyses.*** Annotation of genes was derived from the TIGR Comprehensive Microbial Resource database http://cmr.tigr.org/tigr-scripts/CMR/CmrHomePage.cgi). The computational prediction of subcellular localization of proteins encoded by genes identified in GAF screens was performed using several prediction servers, such as SignalP3.0 (http://www.cbs.dtu.dk/services/SignalP), PSORTb (http://www.psort.org), and TMHMM (http://www.cbs.dtu.dk/services/TMHMM).

### C. EXPERIMENTAL DESIGN

To identify genes essential for the pneumococcus *in vivo,* three independent *Streptococcus pneumoniae* TIGR4 *mariner* transposon mutant libraries of different sizes (approximately 100 (M100), 1,000 (M1000), and 10,000 (M10000) CFU), were used to infect groups of six CD-1 mice in a murine pneumonia model of infection, e.g., a 50 µl-inoculum containing 1x10⁷ CFU administered intranasally. Three mice from each group were sacrificed 24 hours after infection, the remaining three 48 hours after infection, at which time-points nasopharyngeal lavage (NPL), bronchoalveolar lavage (BAL), blood, and lungs (homogenized) were collected. Bacterial load in each sample was determined by plating serial dilutions, and the remainder was stored in 15% glycerol at -80°C. Before DNA isolation and GAF, samples were grown *in vitro* to mid-log phase in GM17 medium supplemented with spectinomycin. GAF analysis of the NPL and blood samples resulted in identification of several mutants that had disappeared from NPL and/or blood during challenge at one or both of the time-points sampled. The corresponding genes can be considered the first potential novel targets identified by *in vivo* GAF, i.e., *Streptococcus pneumonia* genes essential for nasophayngeal colonization and/or dissemination to the blood. These genes are listed in Tables 1A-C and 2A-C

## Claims

1. A vaccine formulation providing protection against pneumococcal infection in a subject, said formulation comprising an effective amount of a protein encoded by a gene listed in Table 1 (1A, 1B and 1C) and/or Table 2 (2A, 2B and 2C) or a functional homologue or an immunogenic part thereof together with at least one of a pharmaceutically acceptable diluent, carrier, excipient or adjuvant therefore.

2. A formulation according to claim 1, wherein said immunogenic part is an antigenic determinant of said pathogen.

3. A formulation according to claim 1 or 2, wherein said protein is encoded by a gene listed in Table 3.

4. A formulation according to claim 1 or 2, wherein said protein is encoded by a gene listed in Table 1A, Table 1B, Table 2A and/or Table 2B.

5. A formulation according to claim 4, wherein said protein is encoded by a gene listed in both Table 1A or Table 1B and Table 2A or Table 2B.

6. A formulation according to any one of claims 1-5, wherein said formulation provides protections against pneumonia, meningitis, otitis media and/or sepsis caused by *Streptococcus pneumoniae.*

7. A protein encoded by a gene listed in Table 1 and/or 2 or an immunogenic part thereof, for use as a vaccine.

8. An antibody against a protein encoded by a gene listed in Table 1 and/or 2 or fragment thereof, preferably a humanized antibody or fragment thereof.

9. An antibody or fragment thereof, preferably a humanized antibody or fragment thereof according to claim 8, for use as a medicament for the prophylactic or therapeutic treatment of a pneumococcal infection in a subject.

10. Use of an antibody or fragment thereof, preferably a humanized antibody or fragment thereof according to claim 8, for the manufacture of a medicament for the prophylactic or therapeutic treatment of a pneumococcal infection in a subject.

11. A pharmaceutical composition comprising an antibody or fragment thereof, preferably a humanized antibody or fragment thereof according to claim 8, and a pharmaceutically acceptable carrier.

12. A method for prophylactic or therapeutic treatment of a pneumococcal infection in a subject comprising administering to a subject in need of such treatment an effective amount of a vaccine formulation as defined in any one of claims 1-6 and/or an effective amount of a pharmaceutical composition as defined in claim 11.

13. A method for preparing an pneumococcal vaccine formulation, the said method comprising bringing into association, an effective amount of a protein encoded by a gene listed in Table 1 and/or Table 2 or an immunogenic part thereof and at least one of a pharmaceutically acceptable diluent, carrier, excipient or adjuvant therefore.

14. A method for preparing a pharmaceutical composition as defined in claim 11, the said method comprising bringing into association, an effective amount of an antibody, preferably a humanized antibody, or fragment thereof, according to claim 8 and a pharmaceutically acceptable carrier.
